# EUROPÄISCHE PATENTSCHRIFT

(11) **EP 1 654 224 B1**
(45) Veröffentlichungstag und Bekanntmachung des Hinweises auf die Patenterteilung: **09.02.2011**
(21) Anmeldenummer: 04763240.1
(22) Anmeldetag: 15.07.2004
(51) Int. Cl.: C07D 205/04, C07D 207/16, C07D 209/42, C07D 211/60, A61K 31/445, A61K 31/397, A61K 31/404, A61P 3/10

(54) **SUBSTITUIERTE BENZOYLUREIDO-O-BENZOYLAMIDE, VERFAHREN ZU DEREN HERSTELLUNG UND DEREN VERWENDUNG**
SUBSTITUTED BENZOYLUREIDO-O-BENZOYLAMIDES, METHOD FOR THE PRODUCTION THEREOF AND USE OF THE SAME
BENZOYLUREIDO-O-BENZOYLAMIDES SUBSTITUES, PROCEDES POUR LEUR PRODUCTION ET LEUR UTILISATION

(30) Priorität: 01.08.2003 DE 10335092
(43) Veröffentlichungstag der Anmeldung: 10.05.2006
(73) Patentinhaber: Sanofi-Aventis Deutschland GmbH, 65929 Frankfurt am Main (DE)
(72) Erfinder: KADEREIT, Dieter, 65926 Frankfurt am Main (DE); DEFOSSA, Elisabeth, 65510 Idstein (DE); SCHOENAFINGER, Karl, 63755 Alzenau (DE); KLABUNDE, Thomas, 65929 Frankfurt (DE); HERLING, Andreas, 65520 Bad Camberg (DE)
(86) Internationale Anmeldenummer: PCT/EP2004/007848
(87) Internationale Veröffentlichungsnummer: WO 2005/012244

(56) Entgegenhaltungen:
- WO-A-01/94300
- WO-A-02/096864
- WO-A-03/104188

## Beschreibung

Die Erfindung betrifft substituierte Benzoylureido-o-benzoylamide sowie deren physiologisch verträgliche Salze und physiologisch funktionelle Derivate.

Es sind bereits strukturähnliche Verbindungen im Stand der Technik sowie deren Verwendung zur Behandlung von Kokainabhängigkeit beschrieben (US 6,121,311).

Es sind bereits Acylphenylharnstoffderivate sowie deren Verwendung zur Behandlung Diabetes beschrieben (WO 001/94300).

Es sind bereits Carbonsäureamid substituierte Phenylharnstoffderivate sowie deren Verwendung zur Behandlung Diabetes beschrieben (WO 02/096864).

Der Erfindung lag die Aufgabe zugrunde, Verbindungen zur Verfügung zu stellen, mit denen eine Prävention und Behandlung von Diabetes Mellitus möglich ist. Die Verbindungen sollten dazu insbesonders eine therapeutisch verwertbare Blutzucker senkende Wirkung entfalten.

Die Erfindung betrifft daher Verbindungen der Formel I, 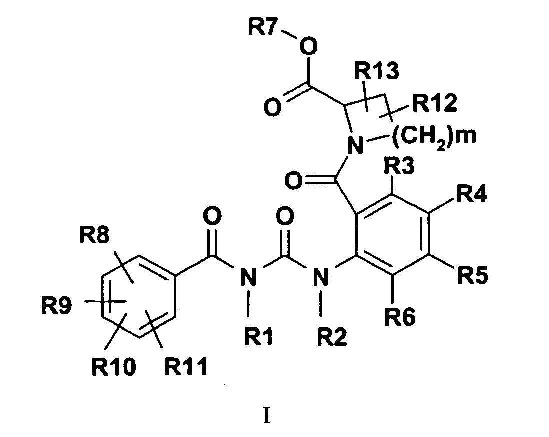 worin bedeuten
- R1, R2: H;
- R3, R4, R5, R6: H, F, Cl, CF₃, COOR14;
- R7: H oder (C₁-C₆)-Alkyl;
- R8, R9: unabhängig von einander F, oder Cl;
- R10, R11: unabhängig von einander H, F, oder Cl;
- R12, R13: unabhängig von einander H oder F; oder
- R12 und R13: bilden gemeinsam mit dem Kohlenstoffatom oder den Kohlenstoffatomen, an die sie gebunden sind, einen 5-6 gliedrigen, gesättigten Ring;
- R14: H oder (C₁-C₄)-Alkyl;
- m: 1, 2, 3;
sowie deren physiologisch verträgliche Salze.

Bevorzugt sind Verbindungen der Formel I, worin ein oder mehrere Reste folgende Bedeutung haben:
- R1, R2: H;
- R3, R4, R5, R6: H;
- R7: H oder (C₁-C₆)-Alkyl;
- R8, R9: unabhängig von einander F, oder Cl;
- R10, R11: unabhängig von einander H, F, oder Cl;
- R12, R13: unabhängig von einander H oder F; oder
- R12 und R13: bilden gemeinsam mit dem Kohlenstoffatom oder den Kohlenstoffatomen, an die sie gebunden sind, einen 5-6 gliedrigen, gesättigten Ring;
- m: 1,2,3;
sowie deren physiologisch verträgliche Salze.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Alkylrest wird eine geradkettige oder verzweigte Kohlenwasserstoffkette mit einem oder mehreren Kohlenstoffen verstanden, wie z.B. Methyl, Ethyl, iso-Propyl, tert.-Butyl, Hexyl.

Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocylus,; PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂,, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)Alkyl, N(C₁-C₆)-Alkyl -COO-(C₁-C₆)-Alkyl, N(C₁₋C₆)-Alkyl-CO-Aryl, N(C₁-C₆)-Alkyl -CO-Heterocyclus, N(C₁-C₆)-Alkyl -COO-Aryl, N(C₁-C6)-Alkyl-COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl-CO-NH-Heterocyclus, N((C₁-C₆)-Alkyl)-CO-N-(C₁-C₆)-Allcyl)₂, N((C₁-C₂)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heteroeyclus)-CO-NH-Aryl, N(Aryl)-CO-N-(C₁-C₆)-Alkyl)₂, N(Heteroeyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocylus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl.

Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,; PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Akyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl-CO-(C₁-C₆)-Alkyl, N(C₁-C₆)-Alkyl-COO-(C₁-C₆)-Alkyl, N(C₁-C₆)-Alkyl-CO-Aryl, N(C₁-C₆)-Alkyl-CO-Heterocyclus, N(C₁-C₆)-Alkyl-COO-Aryl, N(C₁-C₆)-Alkyl - COO-Heterocyclus, N(C₁-C₆)-Alkyl-CO-NH-(C₁-C₆)-Alkyl), N(C₁-C₆)-Alkyl-CO-NH-Aryl, N(C₁-C₆)-Alkyl-CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl; N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C1-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Hexinyl.

Die Alkinylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₁-C₁₀)-Alkyl, O-(C₁-C₆)-Alkyl O-CO-(C₁-C₆)-Alkyl, O-CO-(C₁-C₆)-Aryl, O-CO-(C₁-C₆)-Heteroeyclus; PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂,, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl-CO-(C₁-C₆)-Alkyl, N(C₁-C₆)-Alkyl-COO-(C₁-C₆)-Alkyl, N(C₁-C₆)-Alkyl-CO-Aryl, N(C₁-C₆)-Alkyl-CO-Heterocyclus, N(C₁-C₆)-Alkyl -COO-Aryl, N(C₁-C₆)-Alkyl-COO-Heterocyclus, N(C₁-C₆)-Alkyl-CO-NH-(C₁-C₆)-Alkyl), N(C₁-C₆)-Alkyl-CO-NH-Aryl, N(C₁-C₆)-Alkyl-CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Akyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Akenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,; PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, , SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl-CO-(C₁-C₆)-Alkyl, N(C₁-C₆)-Alkyl-COO-(C₁-C₆)-Alkyl, N(C₁-C₆)-Alkyl - CO-Aryl, N(C₁-C₆)-Alkyl-CO-Heterocyclus, N(C₁-C₆)-Alkyl-COO-Aryl, N(C₁-C₆)-Alkyl - COO-Heterocyclus, N(C₁-C₆)-Alkyl-CO-NH-(C₁-C₆)-Alkyl), N(C₁-C₆)-Alkyl-CO-NH-Aryl, N(C₁-C₆)-Alkyl-CO-NH-Heterocyclus, N((C₁-C₆)-Alkyl)-CO-N-(C₁₋C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heteroeyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylrestereste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,; PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)n-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N(CH₂)ₙ-Aryl)₂,, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl-CO-(C₁-C₆)-Alkyl, N(C₁-C₆)-Alkyl-COO-(C₁-C₆)-Alkyl, N(C₁-C₆)-Alkyl-CO-Aryl, N(C₁-C₆)-Alkyl-CO-Heterocyclus, N(C₁-C₆)-Alkyl -COO-Aryl, N(C₁-C₆)-Alkyl - COO-Heterocyclus, N(C₁-C₆)-Alkyl-CO-NH-(C₁-C₆)-Alkyl), N(C₁-C₆)-Alkyl-CO-NH-Aryl, N(C₁-C₆)-Alkyl-CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryll)₂, N(C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl; CONH₂.

Unter Heterocyclus bzw. Heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der Heterocyclische Rest mit Benzolkernen kondensiert ist.

Geeignete "Heterocyclische Ringe" bzw. "Heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Umfasst sind weiterhin ein oder mehrfach benzoannelierte Derivate dieser Heterozyklen.

Die Heterocyclischen Ringe bzw. Heterocyclische Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)AAlkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus; PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocylus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂,, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl-CO-(C₁-C₆)-Alkyl, N(C₁-C₆)-Alkyl-COO-(C₁-C₆)-Alkyl, N(C₁-C₆)-Alkyl-CO-Aryl, N(C₁-C₆)-Alkyl-CO-Heterocyclus, N(C₁-C₆)-Alkyl-COO-Aryl, N(C₁-C₆)-Alkyl - COO-Heterocyclus, N(C₁-C₆)-Alkyl-CO-NH-(C₁-C₆)-Alkyl), N(C₁-C₆)-Alkyl-CO-NH-Aryl, N(C₁-C₆)-Alkyl-CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindung(en) der Formel (I) können auch in Kombination mit weiteren Wirkstoffen verabreicht werden.

Als weitere Wirkstoffe für die Kombinationspräparate sind geeignet: Alle Antidiabetika, die in der Roten Liste 2003, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen, die in WO 97/26265, WO 99/03861, WO 01/04156, WO 00/34331, WO00/34332, WO91/11457 und US 6,380,357 offenbart wurden, sowie oral wirksame hypoglykämische Wirkstoffe.

Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse-beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit einem PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 00/11833, PCT/US 00/11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide , BMS-201038, R-103757, verabreicht.

Bei einer Ausführungsform der Erfindung werden die Verbindungen der Formel I in Kombination mit einem Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid verabreicht. Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinymethoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., in: Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]- amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2- carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird. 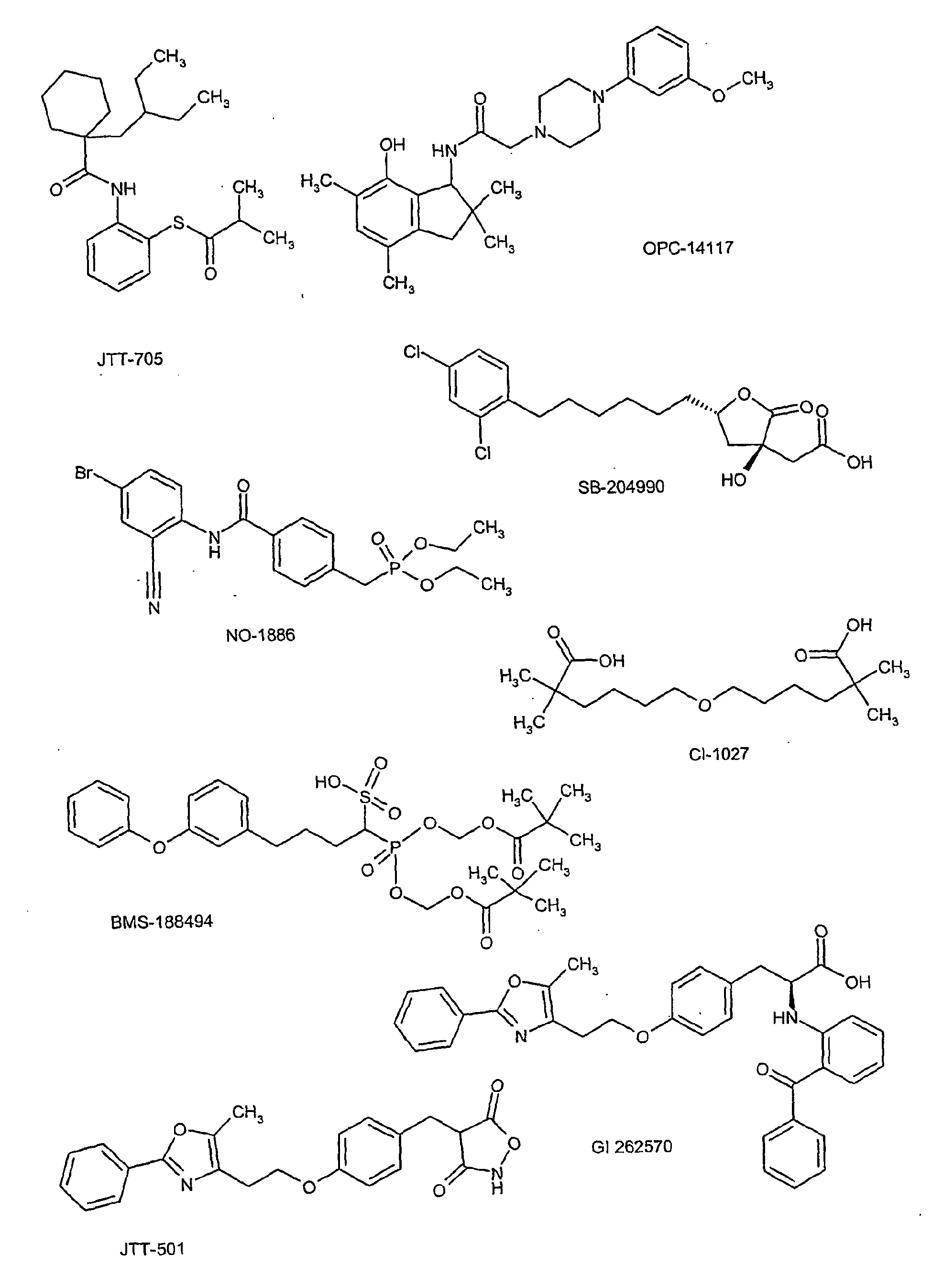 Die nachfolgend aufgeführten Beispiele dienen zur Erläuterung der Erfindung, ohne diese jedoch einzuschränken. Die gemesenen Fest-, bzw. Zersetzungspunkte (Fp.) wurden nicht korrigiert und sind generell von der Aufheizgeschwindigkeit abhängig.

| **Bsp.** | **Struktur** | **Fp.** [°C] |
|---|---|---|
| 1 | 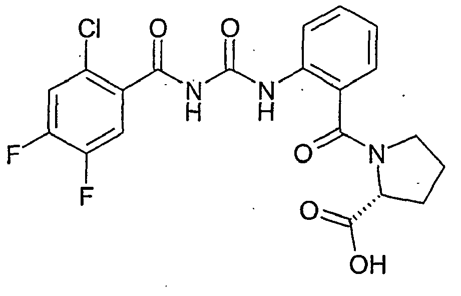 | 123,5 |
| 2 | 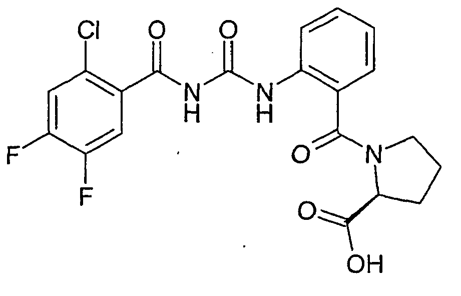 | 128,4 |
| 3 | 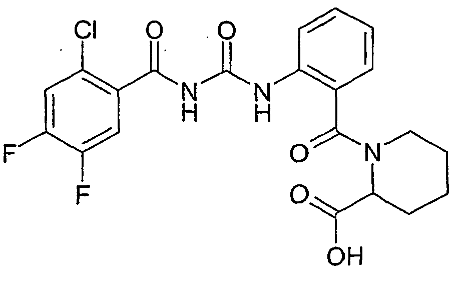 | 193,1 |
| 4 | 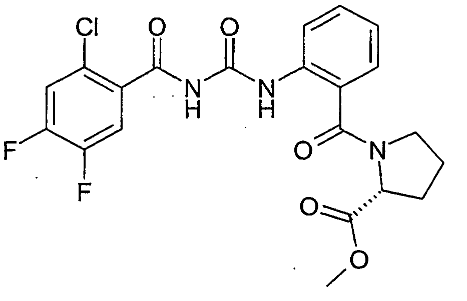 | Öl |
| 5 | 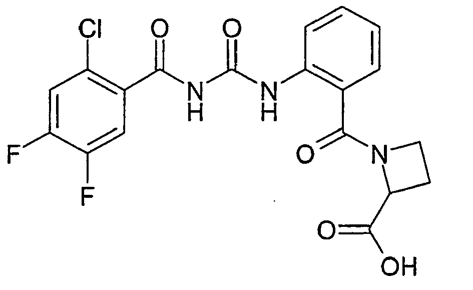 | 99 (Erweichung) |
| 6 | 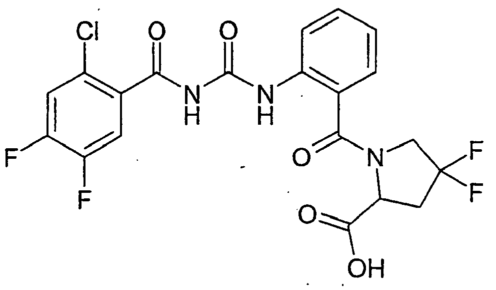 | 102 (Erweichung) |
| 7 | 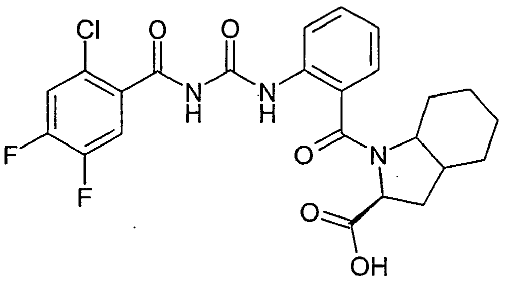 | 125 (Erweichung) |

Die Wirksamkeit der Verbindungen wurde wie folgt getestet:

### Glykogenphosphorylase a Aktivitätstest

Der Effekt von Verbindungen auf die Aktivität der aktiven Form der Glykogenphosphorylase (GPa) wurde in der umgekehrten Richtung, durch Verfolgen der Glykogensynthese aus Glukose-1-Phosphat an Hand der Bestimmung der Freisetzung von anorganischem Phosphat, gemessen. Alle Reaktionen wurden als Doppelbestimmungen in Mikrotiterplatten mit 96-Vertiefungen (Half Area Plates, Costar Nr. 3696) durchgeführt, wobei die Änderung der Absorption auf Grund der Bildung des Reaktionsprodukts bei der weiter unten spezifizierten Wellenlänge in einem Multiskan Ascent Elisa Reader (Lab Systems, Finnland) gemessen wurde.

Um die GPa Enzymaktivität in der umgekehrten Richtung zu messen, wurde die Umwandlung von Glukose-1-Phosphat in Glykogen und anorganisches Phosphat nach der allgemeinen Methode von Engers et al. (Engers HD, Shechosky S, Madsen NB, Can J Biochem 1970 Jul;48(7):746-754) mit folgenden Modifikationen gemessen: Humane Glykogenphosphorylase a (zum Beispiel mit 0,76 mg Protein / ml (Aventis Pharma Deutschland GmbH), gelöst in Pufferlösung E (25 mM β-Glyzerophosphat, pH 7,0, 1 mM EDTA und 1 mM Dithiotreitol) wurde mit Puffer T (50 mM Hepes, pH 7,0, 100 mM KCl, 2,5 mM EDTA, 2,5 mM MgCl₂·6H₂O) und Zusatz von 5 mg/ml Glykogen auf eine Konzentration von 10 µg Protein/ml verdünnt. Prüfsubstanzen wurden als 10 mM Lösung in DMSO zubereitet und auf 50 µM mit Pufferlösung T verdünnt. Zu 10 µl dieser Lösung wurden 10 µl 37,5 mM Glukose, gelöst in Pufferlösung T und 5 mg/mL Glykogen, sowie 10 µl einer Lösung von humaner Glykogenphosphorylase a (10 µg Protein/ml) und 20 µl Glukose-1-Phosphat, 2,5 mM zugegeben. Der basale Wert der Glykogenphosphorylase a Aktivität in Abwesenheit von Prüfsubstanz wurde durch Zugabe von 10 µl Pufferlösung T (0,1 % DMSO) bestimmt. Die Mischung wurde 40 Minuten bei Raumtemperatur inkubiert und das freigesetzte anorganische Phosphat mittels der allgemeinen Methode von Drueckes et al. (Drueckes P, Schinzel R, Palm D, Anal Biochem 1995 Sep 1;230(1):173-177) mit folgenden Modifikationen gemessen: 50 µl einer Stop-Lösung von 7,3 mM Ammoniummolybdat, 10,9 mM Zinkacetat, 3,6 % Askorbinsäure, 0,9 % SDS werden zu 50 µl der Enzymmischung gegeben. Nach 60 Minuten Inkubation bei 45 °C wurde die Absorption bei 820 nm gemessen. Zur Bestimmung der Hintergrundsabsorption wurde in einem separaten Ansatz die Stop-Lösung unmittelbar nach Zugabe der Glukose-1-Phosphatlösung zugegeben.

Dieser Test wurde mit einer Konzentrationen von 10 µM der Prüfsubstanz durchgeführt, um die jeweilige Hemmung der Glykogenphosphorylase a in vitro durch die Prüfsubstanz zu bestimmen.

**Tabelle 2: Biologische Aktivität**

| **Bsp.** | **IC-50 (µM)** |
|---|---|
| 1 | 0,03 |
| 2 | 2,1 |
| 3 | 0,12 |
| 4 | 0,57 |
| 5 | 0,03 |
| 6 | 1,4 |
| 7 | 4,0 |

Aus der Tabelle ist abzulesen, dass die Verbindungen der Formel I die Aktivität der Glykogenphosphorylase a hemmen und dadurch zur Senkung des Blutzuckerspiegels gut geeignet sind. Sie eignen sich damit insbesonders zur Behandlung von Diabetes Typ I und II, von Insulinresistenz, von Dyslipidämien, des metabolischen Syndroms / Syndrom X, von krankhafter Fettleibigkeit und zur Gewichtsreduktion bei Säugetieren.

Weiterhin eignen sich Verbindungen der Formel I, wegen ihrer Hemmung der PTP1B, zur Behandlung von Hyperglycerimia, Bluthochdruck, Atherosklerose, Fehlfunktionen des Immunsystems, Autoimmunkrankheiten, allergischen Krankheiten wie z.B. Asthma, Arthritis, Osteoarthritis, Osteoporose, Proliferationsstörungen wie Krebs und Psoriasis, Krankheiten mit verminderter oder erhöhter Produktion von Wachstumsfaktoren, Hormonen oder Cytokinen, die die Freisetzung von Wachstumshormonen auslösen.

Die Verbindungen eignen sich auch zur Behandlung Erkrankungen des Nervensystems, wie zum Beispiel Alzheimer oder Multiple Sklerose.

Die Verbindungen eignen sich auch zur Behandlung von Störungen des Empfindens und anderer psychiatrischen Indikationen, wie zum Beispiel Depressionen, Angstzuständen, Angstneurosen, Schizophrenie, zur Behandlung von Störungen assoziiert mit dem zirkadianen Rhythmus und zur Behandlung von Drogenmissbrauch.

Weiterhin eignen sie sich zur Behandlung von Schlafstörungen, Schlaf Apnoe, weiblicher und männlicher Sexualstörungen, Entzündungen, Akne, Pigmentierung der Haut, Störungen des Steroidstoffwechsels, Hautkrankheiten und Mykosen.

Nachfolgend wird die Herstellung eines Beispiels detailliert beschrieben, die übrigen Verbindungen der Formel I wurden analog erhalten:

### Experimenteller Teil:

### Beispiel 1:

### R-1-{2-[3-(2-Chlor-4,5-difluor-benzoyl)-ureido]-benzoyl}-pyrrolidin-2-carbonsäure

Zur Lösung von 0,2 g Isatosäureanhydrid in 5 ml Acetonitril werden 0,148 g D-Prolin und 0,2 ml Triethylamin zugefügt und die Mischung 2 Stunden bei Raumtemperatur gerührt. Dann werden 1,3 mmol einer Lösung von 2-Chlor-4,5-difluor-benzoyl-isocyanat in Acetonitril zugetropft und 30 Minuten bei Raumtemperatur weitergerührt. Nach dem Abziehen der flüchtigen Anteile im Vakuum bei 40°C wird der Rückstand mit 1N Salzsäure 15 Stunden lang verrührt, der gebildete Niederschlag abgesaugt und im Vakuum getrocknet.

Ausbeute: 0,61 g Fp.: 123,5 °C

Die weiteren Beispiele wurden analog hergestellt.

## Patentansprüche

1. Verbindungen der Formel I, 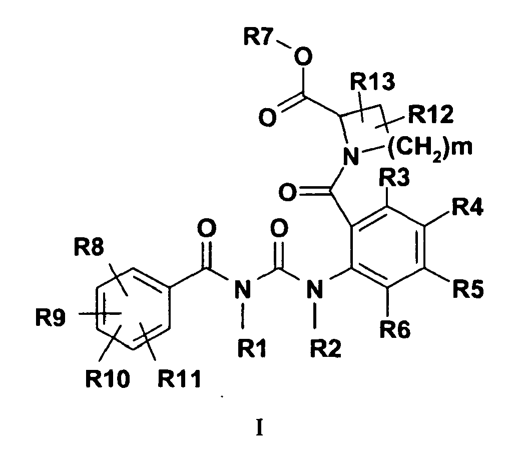 worin bedeuten
R1, R2 H;
R3, R4, R5, R6 H, F, Cl, CF₃, COOR14;
R7 H oder (C₁-C₆)-Alkyl;
R8, R9 unabhängig von einander F, oder Cl;
R10, R11 unabhängig von einander H, F, oder Cl;
R12, R13 unabhängig von einander H oder F; oder
R12 und R13 bilden gemeinsam mit dem Kohlenstoffatom oder den Kohlenstoffatomen, an die sie gebunden sind, einen 5-6 gliedrigen, gesättigten Ring;
R14 H oder (C₁-C₄)-Alkyl;
m 1,2,3;
sowie deren physiologisch verträgliche Salze.

2. Verbindungen gemäß Anspruch 1, **dadurch gekennzeichnet, dass** darin bedeuten
R1, R2 H;
R3, R4, R5, R6 H;
R7 H oder (C₁-C₆)-Alkyl;
R8, R9 unabhängig von einander F, oder Cl;
R10, R11 unabhängig von einander H, F, oder Cl;
R12, R13 unabhängig von einander H oder F; oder
R12 und R13 bilden gemeinsam mit dem Kohlenstoffatom oder den Kohlenstoffatomen, an die sie gebunden sind, einen 5-6 gliedrigen, gesättigten Ring;
m 1, 2, 3;
sowie deren physiologisch verträgliche Salze.

3. Verbindungen gemäß einem oder mehreren der Ansprüche 1 und 2 zur Anwendung als Arzneimittel.

4. Arzneimittel enthaltend eine oder mehrere der Verbindungen gemäß einem oder mehreren der Ansprüche 1 und 2.

5. Arzneimittel enthaltend eine oder mehrere der Verbindungen gemäß einem oder mehreren der Ansprüche 1 und 2 und mindestens einen weiteren Wirkstoff.

6. Arzneimittel, gemäß Anspruch 5, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylhamstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2-oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

7. Verwendung der Verbindungen gemäß einem oder mehreren der Ansprüche 1 und 2 zur Herstellung eines Medikamentes zur Blutzuckersenkung.

8. Verwendung der Verbindungen gemäß einem oder mehreren der Ansprüche 1 und 2 zur Herstellung eines Medikamentes zur Behandlung des Typ II Diabetes.

9. Verwendung der Verbindungen gemäß einem oder mehreren der Ansprüche 1 und 2 zur Herstellung eines Medikamentes zur Behandlung von Lipid- und Kohlenhydratstoffwechselstörungen.

10. Verwendung der Verbindungen gemäß einem oder mehreren der Ansprüche 1 und 2 zur Herstellung eines Medikaments zur Behandlung arteriosklerotischer Erscheinungen.

11. Verwendung der Verbindungen gemäß einem oder mehreren der Ansprüche 1 und 2 zur Herstellung eines Medikaments zur Behandlung von Insulin Resistenz.

12. Verfahren zur Herstellung eines Arzneimittels enthaltend eine oder mehrere der Verbindungen gemäß einem oder mehreren der Ansprüche 1 und 2, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I, 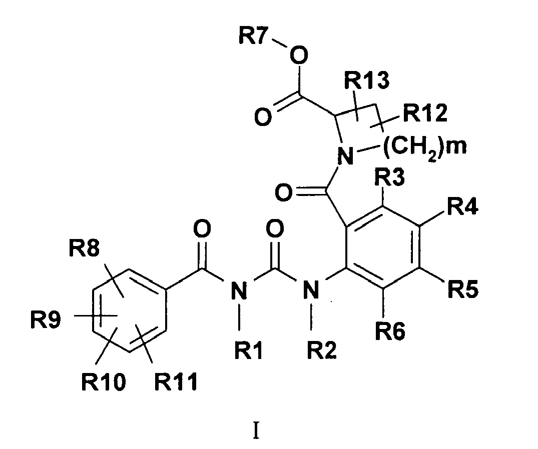 in which the meanings are
R1, R2 H;
R3, R4, R5, R6 H, F, Cl, CF₃, COOR14;
R7 H or (C₁-C₆)-alkyl;
R8, R9 independently of one another F, or Cl;
R10, R11 independently of one another H, F, or Cl;
R12, R13 independently of one another H or F; or
R12 and R13 form together with the carbon atom or the carbon atoms to which they are bonded a 5-6 membered, saturated ring;
R14 H or (C₁-C₄)-alkyl;
m 1,2,3;
and the physiologically tolerated salts thereof.

2. The compound as claimed in claim 1, wherein the meanings are
R1, R2 H;
R3, R4, R5, R6 H;
R7 H or (C₁-C₆)-alkyl;
R8, R9 independently of one another F, or Cl;
R10, R11 independently of one another H, F, or Cl;
R12, R13 independently of one another H or F; or
R12 and R13 form together with the carbon atom or the carbon atoms to which they are bonded a 5-6 membered, saturated ring;
m 1,2,3;
and the physiologically tolerated salts thereof.

3. The compound as claimed in one or more of claims 1 and 2 for use as medicament.

4. A medicament comprising one or more of the compounds as claimed in one or more of claims 1 and 2.

5. A medicament comprising one or more of the compounds as claimed in one or more of claims 1 and 2 and at least one other active ingredient.

6. The medicament as claimed in claim 5, wherein the other active ingredient comprises one or more antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed sertoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

7. The use of the compounds as claimed in one or more of claims 1 and 2 for producing a medicament for reducing blood glucose.

8. The use of the compounds as claimed in one or more of claims 1 and 2 for producing a medicament for the treatment of type II diabetes.

9. The use of the compounds as claimed in one or more of claims 1 and 2 for producing a medicament for the treatment of disturbances of lipid and carbohydrate metabolism.

10. The use of the compounds as claimed in one or more of claims 1 and 2 for producing a medicament for the treatment of arteriosclerotic manifestations.

11. The use of the compounds as claimed in one or more of claims 1 and 2 for producing a medicament for the treatment of insulin resistance.

12. A process for producing a medicament comprising one or more of the compounds as claimed in one or more of claims 1 and 2, which comprises mixing the active ingredient with a pharmaceutically suitable carrier and converting this mixture into a form suitable for administration.

## Revendications

1. Composés de formule I, 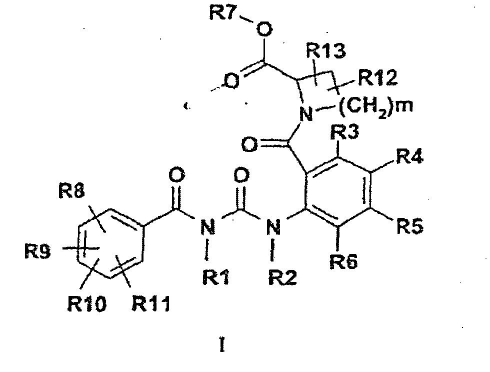 où
R1, R2 signifient H ;
R3, R4, R5, R6 signifient H, F, Cl, CF₃, COOR14 ;
R7 signifie H ou (C₁-C₆) -alkyle ;
R8, R9 indépendamment l'un de l'autre, signifient F ou Cl ;
R10, R11 indépendamment l'un de l'autre, signifient H, F ou Cl ;
R12, R13 indépendamment l'un de l'autre, signifient H ou F ; ou
R12 et R13 forment ensemble avec l'atome de carbone ou les atomes de carbone au(x)quel(s) ils sont liés un cycle saturé de 5-6 chaînons ;
R14 signifie H ou (C₁-C₄)-alkyle ;
m vaut 1, 2, 3 ;
ainsi que leurs sels physiologiquement acceptables.

2. Composés selon la revendication 1, **caractérisés en ce que**
R1, R2 signifient H ;
R3, R4, R5, R6 signifient H ;
R7 signifie H ou (C₁-C₆)-alkyle ;
R8, R9 indépendamment l'un de l'autre, signifient F ou Cl ;
R10, R11 indépendamment l'un de l'autre, signifient H, F ou Cl ;
R12, R13 indépendamment l'un de l'autre, signifient H ou F ; ou
R12 et R13 forment ensemble avec l'atome de carbone ou les atomes de carbone au (x) quel (s) ils sont liés un cycle saturé de 5-6 chaînons ;
m vaut 1, 2; 3 ;
ainsi que leurs sels physiologiquement acceptables.

3. Composés selon l'une ou plusieurs des revendications 1 et 2 destinés à être utilisés comme médicament.

4. Médicament contenant un ou plusieurs des composés selon l'une ou plusieurs des revendications 1 et 2.

5. Médicament contenant un ou plusieurs des composés selon l'une ou plusieurs des revendications 1 et 2 et au moins une autre substance active.

6. Médicament selon la revendication 5, **caractérisé en ce qu'**il contient comme autre substance active un(e) ou plusieurs antidiabétiques, substances actives hypoglycémiques, inhibiteurs de la HMGCoA-réductase, inhibiteurs de la résorption de cholestérol, agonistes de PPAR gamma, agonistes de PPAR alpha, agonistes de PPAR alpha/gamma, fibrates, inhibiteurs de MTP, inhibiteurs de la résorption de l'acide biliaire, inhibiteurs de CETP, adsorbants polymères de l'acide biliaire, inducteurs de récepteurs de LDL, inhibiteurs d'ACAT, antioxydants, inhibiteurs de la lipoprotéine-lipase, inhibiteurs de l'ATP-citrate lyase, inhibiteurs de la squalène-synthétase, antagonistes de la lipoprotéine (a), inhibiteurs de lipase, insulines, sulfonylurées, biguanides, méglitinides, thiazolidinediones, inhibiteurs de l'α-glucosidase, substances actives agissant sur le canal potassique dépendant d'ATP des cellules bêta, agonistes de CART, antagonistes de NPY, agonistes de MC4, agonistes d'orexine, agonistes de H3, agonistes de TNF, agonistes de CRF, antagonistes de CRF BP, agonistes d'urocortine, agonistes de β3, agonistes de MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, agonistes de galanine, hormones de croissance, composés libérant l'hormone de croissance, agonistes de TRH, modulateurs 2 ou 3 de la protéine découplante, agonistes de leptine, agonistes de DA (Bromocriptine, Doprexine), inhibiteurs de lipase/amylase, modulateurs de PPAR, modulateurs de RXR ou agonistes de TR-β ou amphétamines.

7. Utilisation des composés selon l'une ou plusieurs des revendications 1 et 2 pour la préparation d'un médicament destiné à abaisser la glycémie.

8. Utilisation des composés selon l'une ou plusieurs des revendications 1 et 2 pour la préparation d'un médicament destiné au traitement du diabète de type II.

9. Utilisation des composés selon l'une ou plusieurs des revendications 1 et 2 pour la préparation d'un médicament destiné au traitement des troubles du métabolisme lipidique et des hydrates de carbone.

10. Utilisation des composés selon l'une ou plusieurs des revendications 1 et 2 pour la préparation d'un médicament destiné au traitement de phénomènes artérioscléreux.

11. Utilisation des composés selon l'une ou plusieurs des revendications 1 et 2 pour la préparation d'un médicament destiné au traitement de la résistance à l'insuline.

12. Procédé pour la préparation d'un médicament contenant un ou plusieurs des composés selon l'une ou plusieurs des revendications 1 et 2, **caractérisé en ce que** la substance active est mélangée avec un support pharmaceutiquement approprié et ce mélange est amené dans une forme appropriée pour l'administration.
